Europäisches Patentamt

European Patent Office

Office européen des brevets

(19)

(11) Veröffentlichungsnummer: **0 336 983**
**A1**

(12)

# EUROPÄISCHE PATENTANMELDUNG
Veröffentlicht nach Art. 158 Abs. 3 EPÜ

(21) Anmeldenummer: 88909006.4

(22) Anmeldetag: 16.09.88

Daten der zugrundeliegenden internationalen Anmeldung:

(86) Internationale Anmeldenummer:
PCT/SU88/00179

(87) Internationale Veröffentlichungsnummer:
WO89/02253 (23.03.89 89/07)

(51) Int. Cl.³: **A 61 F 2/24**
**A 61 L 27/00**

(30) Priorität: 18.09.87 SU 4303285

(43) Veröffentlichungstag der Anmeldung:
18.10.89 Patentblatt 89/42

(84) Benannte Vertragsstaaten:
BE DE GB IT LU NL SE

(71) Anmelder: BUKATOV, Alexandr Semenovich
pl. Pobedy, 1-A-122
Moscow, 121293(SU)

(71) Anmelder: KOROTEEVA, Irina Viktorovna
Frunzenskaya nab. 50-223
Moscow, 119270(SU)

(71) Anmelder: IOFIS, Naum Abramovich
Lomonosovsky pr. 23-416
Moscow, 117311(SU)

(71) Anmelder: KOSTRETSOV, Anatoly Stepanovich
ul. Bakuninskaya, 10/12-7
Moscow, 107005(SU)

(71) Anmelder: PRISTAVKO, Evgeny Gerasimovich
ul. Zveyas, 38-1
Riga 226030(SU)

(72) Erfinder: BUKATOV, Alexandr Semenovich
pl. Pobedy, 1-A-122
Moscow, 121293(SU)

(72) Erfinder: KOROTEEVA, Irina Viktorovna
Frunzenskaya nab. 50-223
Moscow, 119270(SU)

(72) Erfinder: IOFIS, Naum Abramovich
Lomonosovsky pr. 23-416
Moscow, 117311(SU)

(72) Erfinder: KOSTRETSOV, Anatoly Stepanovich
ul. Bakuninskaya, 10/12-7
Moscow, 107005(SU)

(72) Erfinder: PRISTAVKO, Evgeny Gerasimovich
ul. Zveyas, 38-1
Riga 226030(SU)

(74) Vertreter: von Füner, Alexander, Dr. et al,
Patentanwälte v. Füner, Ebbinghaus, Finck Mariahilfplatz
2 & 3
D-8000 München 90(DE)

(54) **TITANIMPLANTAT FÜR KARDIOVASKULARE CHIRURGIE UND VERFAHREN ZUR HERSTELLUNG.**

(57) Das Titanimplantat weist plastisch verformte Oberflächen, die unter Einwirkung von freien granulierten Körpern in Form von Kugeln verdichtet sind. Solche Oberflächenstruktur wird durch die Bearbeitung des Werkstücks für Implantat in einer Einrichtung für die Bearbeitung im Planeten-Schleuderverfahren erreicht, in welcher man das Werkstück in eine mit Kugeln und wässriger Seifenlösung angefüllte Kammer einbringt, wodurch im Laufe der Rotation der Kammer beim Aufprallen der Kugeln mit Werkstückoberflächen die Bearbeitung derselben erfolgt, die die erforderliche Oberflächenstruktur ergibt. Das hergestellte Implantat besitzt eine erhöhte Thrombenresistenz und hohe biologische Trägheit.

EP 0 336 983 A1

# TITANIMPLANTAT FÜR DIE KARDIOVASKULÄRE CHIRURGIE UND VERFAHREN ZUR HERSTELLUNG DESSELBEN

## Gebiet der Technik

Die vorliegende Erfindung bezieht sich auf die Medizin und insbesondere auf ein Implantat für die kardiovaskuläre Chirurgie und ein Verfahren zur Herstellung desselben.

## Zugrundeliegender Stand der Technik

In den letzten Jahren finden Metallimplantate, insbesondere aus Kobalt und Titan in der Kardiochirurgie zunehmend Anwendung, wobei die Titanimplantate wegen hoher Kobaltkosten am meisten verbreitet sind. Als Implantate dienen in der Kardiochirurgie weitestgehend Herzklappanprothesen, welche einen Ring mit der Öffnung zum Durchfliessen der Blut enthalten, in der ein Sperrglied (US, A, 4713071) angebracht ist. An solche Herzklappenprothese werden harte Anforderungen hinsichtlich der Vermeidung der Thrombenbildung unter Einwirkung ihrer Oberfläche sowie hinsichtlich der äusserst geringen biologischen Wirksamkeit oder hohen biologischen Trägheit der Oberflächen gestellt.

Zur Erfüllung dieser hohen Anforderungen an die Oberfläche wurde schon vorgeschlagen, auf die vorbehandelte Oberfläche oder auf die vorbehandelten Oberflächen verschiedene Überzüge, insbesondere einen Überzug aus spektroskopisch reinem Kohlenstoff (SU, A, 1155263) aufzubringen. Die dabei herstellbare Herzklappenprothese weist auf ihrer Oberfläche die hochwertige 6 bis 8 $\mu$m dicke Kohlenstoffschicht auf, die die Thrombenbildung in ihrem Ausgangszustand wirklich verhindert und eine sehr geringe biologische Wirksamkeit besitzt. Dieses Verfahren hat inzwischen wegen einer ihm eigenen Reihe von Nachteilen keine Verwendung gefunden, wobei von denen folgende zu nennen sind: Es ist sehr schwer, eine gleichmässige Dicke über die ganze Oberfläche der Herzklappe zu erreichen, und der Dickenunterschied verschlechtert das hämodynamische Stromverhalten. In diesem Falle

- 2 -

fordert das Überziehen die Verwendung von komplizierten und kostspieligen Einrichtungen, um das Hochvakuum von etwa $10^{-6}$ Torr zu erzeugen und die Temperaturen genau zu kontrollieren. Und schliesslich beginnt sich der herstellbare Überzug mit der Zeit abzuschichten und zu schälen, und dies führt zu sehr unangenehmen Folgen unter besonderer Berücksichtigung der Unmöglichkeit, die Reparaturarbeiten am anwendbaren Implantat durchzuführen. Gerade infolge der angegebenen Verhältnisse kommt sowohl dieses Verfahren als auch eine Reihe von anderen Verfahren heute zur Verwendung nicht.

In der Praxis werden verschiedene modernisierte schon bekannte Verfahren benutzt, welche den Einsatz von Titanimplantaten vorsehen, die keinen Überzug, aber die hochwertige Oberfläche besitzen, wobei die hohe Oberflächengüte durch vielstufige Bearbeitung einschliesslich solcher Arbeitsgänge wie Schleifen, Polieren, Läppolieren mit Pasten und andere Arten der Sonderbearbeitung erreicht wird, welche die Herstellung der Oberfläche mit einer Rauheit von etwa 0,16 $\mu$m sichern, was der Meinung von Fachleuten nach die hohe Thrombenresistenz ergeben soll. Wie die Praxis gezeigt hat, beeinflusst inzwischen die so hohe Bearbeitungsgüte die Thrombenresistenz entscheidend nicht und zugleich erweist sich dieser vielstufige Prozess als dauernd und teuer. Es sei dabei berücksichtigt werden, dass der Grundfaktor, nämlich die Neigung des Implantats zur Thrombenbildung durch Vorhandensein einer porigen Struktur innerhalb seiner Oberfläche bedingt ist, die im Laufe der konventionellen Bearbeitung erhalten bleibt.

Es ist also anerkennenswert, dass es einen Bedarf gibt, eine prinzipiell neue Einstellung zur Entwicklung von Implantaten für die kardiovaskuläre Chirurgie zu erarbeiten, die eine einfache, aber zugleich sehr thrombenresistente Oberflächenstruktur aufweisen, welche nach einem einfachen Verfahren hergestellt werden könnte.

- 3 -

## Offenbarung der Erfindung

Der Erfindung liegt die Aufgabe zugrunde, die Oberflächen eines Titanimplantats so auszuführen, dass sie solch eine Struktur besitzen, die eine erhöhte Thrombenresistenz und erniedrigte biologische Wirksamkeit sichert, sowie ein einfaches und wirtschaftliches Verfahren zur Herstellung solcher Schichten an Implantaten zu entwickeln.

Die gestellte Aufgabe wird dadurch gelöst, dass beim Titanimplantat für die kardiovaskuläre Chirurgie, welches bearbeitete thrombenresistente biologisch träge Oberflächen aufweist, bearbeitete Oberflächen erfindungsgemäss plastisch verformte und unter Einwirkung von freien granulierten Körpern verdichtete Oberflächen darstellen.

Der Vorteil solcher Oberflächenstruktur liegt darin, dass sie keine dem gewöhnlichen Titan eigenen Poren aufweist, wodurch die Möglichkeit der Thrombenbildung bedeutend herabgesetzt wird, wobei die von uns durchgeführten Versuche, wie ferner gezeigt werden wird, auch eine äusserst geringe biologische Wirksamkeit des Implantats mit solcher Oberfläche ergeben haben.

Diese Oberflächenstruktur des Implantats kann nach einem Verfahren hergestellt werden, welches die Vor- und Nachbearbeitung des Werkstücks vorsieht, wobei man erfindungsgemäss im Stadium der Nachbearbeitung das Werkstück in eine mit polierten Granalien als Rotationskörpern und wässriger Seifenlösung eingefüllte Kammer einbringt und die Kammer in Planetenbewegung mit einer Geschwindigkeit versetzt, die ausreicht, um die plastische Verformung von Oberflächen des Werkstücks durch Zusammenwirken derselben mit Granalien auszulösen.

Der Vorteil dieses Verfahrens besteht darin, dass die hohe Feinheit der bearbeiteten Oberfläche und praktisch das volle Fahlen von Poren innerhalb der Titanoberfläche erreicht werden, wodurch die hohe Thrombenresistenz gesichert wird. Die Verwendung dieses Verfahrens macht es ausserdem möglich, die Anforderungen an

- 4 -

die Vorbearbeitungsgüte herabzusetzen, weil die erreichbare Oberflächenfeinheit praktisch in einem sehr geringen Masse von der Vorbearbeitung abhängt.

Die Granalien können beliebige Rotationskörper darstellen, aber am zweckmässigsten ist die Verwendung von Kugeln, weil es in diesem Fall zum Punktkontakt mit der zu bearbeitenden Oberfläche und zur besten Verdichtung der Oberfläche durch plastische Verformung derselben kommt.

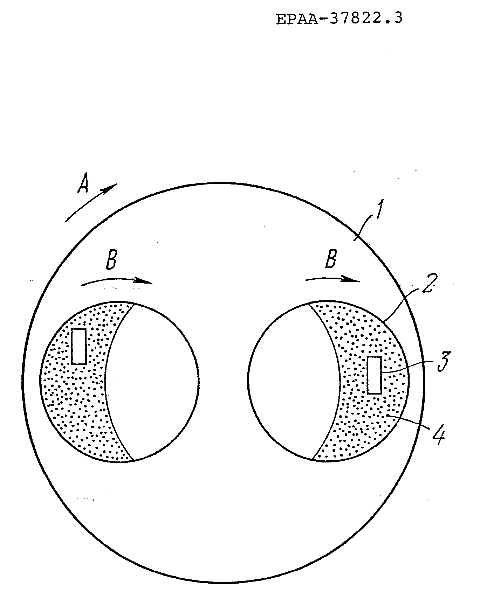

Kurze Beschreibung der Zeichnung

Nachstehend wird die Erfindung an Hand der Beschreibung der konkreten Ausführungsform und der beiliegenden Zeichnung näher erläutert, wobei die Zeichnung das Schema der Einrichtung zur Durchführung des Verfahrens zur Herstellung der plastisch verformten Oberfläche des Implantats darstellt.

Die beste Ausführungsform der Erfindung

Ein Implantat für die kardiovaskuläre Chirurgie, welches eine plastisch verformte Oberflächenschicht oder einfach eine plastisch verformte Oberfläche besitzt, kann mit Hilfe einer in der Zeichnung gezeigten Einrichtung hergestellt werden. Diese Einrichtung enthält einen nach Pfeil A drehbar angeordneten Rotor 1, in dem zwei Kammern 2 untergebracht sind, die um ihre eigene Achse nach dem Pfeil B und zusammen mit dem Rotor 1 drehbar angeordnet sind. Ein Werkstück 3 für Implantat wird in die Kammer 2 eingebracht, die mit darin frei liegenden Granalien 4 und mit der darin einzugiessenden wässrigen Seifenlösung eingefüllt wird. Granalien 4 sollen als Rotationskörper ausgeführt sein, um den Punkt- oder Linienkontakt mit dem zu bearbeitenden Werkstück 3 für Implantat sicherzustellen. Beim gleichzeitigen Rotieren des Rotors 1 und der Kammern 2, wie die Pfeile zeigen, gewinnen die in den Kammern befindlichen Werkstücke 3 und Granalien 4 an kinetischer Energie, prallen aufeinander, wodurch die plastische Verformung der Oberfläche des Werkstücks 3 für Implantat erfolgt, so dass die durch Nachbearbeitung her-

stellbare Oberfläche glatt und blank ist. Die bearbeitete Oberfläche weist keine Poren auf, was sich günstig auf der Thrombenresistenz auswirkt und sie praktisch ausschliesst.

Obwohl nach dem beschriebenen Verfahren unterschiedliche kardiochirurgische Implantate einschliesslich Herzklappen, Röhrenelemente für Gefässe und anderes mehr hergestellt werden können, ist die Bearbeitung von Herzklappenprothesen und zwar ihres Gehäuses und Sperrgliedes von grösstem Interesse. Betrachten wir einige Beispiele für die Bearbeitung der Gehäuse von Herzklappenprothesen. Man stellte zuerst Werkstücke für Gehäuse aus Reintitan her. Jedes Gehäuse wurde vorbearbeitet, um die bestimmte gegebene Oberflächenfeinheit zu erreichen, und dann entfettet. Nach dieser Vorbearbeitung wurde das Werkstück an Hand der nachstehenden Beispiele gemäss der vorliegenden Erfindung bearbeitet.

Beispiel 1

Ein vorbearbeitetes Gehäuse der Herzklappenprothese wurde in die 1 Liter grosse Kammer einer Einrichtung zur Bearbeitung in einem Planeten-Schleuderverfahren eingebracht, wobei diese Einrichtung vom Experimentier-Forschungsinstitut für Werkzeugmaschinen (Eksperimentalny nauchno-issledovatelsky institut metallorezhuschikh stankov ENIIMS (Moskau) gefertigt wird und aus diesem zum Verkauf kommt. Diese Kammer wurde zu 50% Volumen mit einem Gemisch von polierten gehärteten Chromstahlkugeln mit einem Durchmesser von 0,8 bis 1,0 mm angefüllt, welche die Bearbeitung aller Abschnitte der Werkstückfläche ausführen. In die Kammer wurde dann 0,5 Liter wässrige Seitenlösung eingegossen. Die Kammern wurden in Planetenbewegung versetzt, wobei die Winkelgeschwindigkeiten der Translation und Relativdrehung 60 U/min betrugen. Die Bearbeitung dauerte 3 Stunden. Die bearbeiteten Werkstücke unterlagen der Sichtprüfung und Beurteilung der Oberflächenrauhigkeit, wobei die Rauhtiefe $R_a$ bei 0,32 $\mu$m lag, die Oberflächenporen waren nicht vorhanden.

- 6 -

Wir haben die Versuche mit einer geringeren Relativdrehgeschwindigkeit bei den gleichen Kugelgrössen durchgeführt. Die Oberfläche wurde trübe, eine bedeutende Porenmenge blieb erhalten.

Beispiel 2

Die Verfahrensweise des Beispiels 1 wurde wiederholt, nur dass die Geschwindigkeit 150 U/min betrug. Das Gehäuse hatte eine blanke Oberfläche, die Poren fehlten. Die Rauhtiefe $R_a$ betrug 0,20 $\mu$m.

Beispiel 3

Die Verfahrensweise des Beispiels 1 wurde wiederholt, nur dass die Winkelgeschwindigkeit 200 U/min betrug. Das Gehäuse hatte eine helle glänzende Oberfläche. Die Rauhtiefe $R_a$ betrug 0,18 $\mu$m. In demselben Versuch wurde ein zusätzliches Paar von Gehäusen mit einer Geschwindigkeit von mehr als 200 U/min bearbeitet. Die dabei hergestellten Gehäuse wiesen jedoch den Grat am Rand bearbeiteten Oberflächen wegen zu starker plastischer Verformung auf.

Zu Zwecken der Bestimmung der biologischen Trägheit von hergestellten Erzeugnissen wurden von uns Titanplatten unter Bedingungen gemäss Beispielen 1 bis 3 zusätzlich bearbeitet. Diese Titanplatten wurden danach in Simulationsmedien (physiologische Lösung, Plasma und Blutserum) bei einer Konstanttemperatur von 37°C innerhalb von 1 Jahr geprüft. Durch die elektronenmikroskopische Untersuchung dieser Platten waren keine Mikrofehler festgestellt, während die Platten aus ähnlichem Titan, die wie oben angegeben nicht bearbeitet wurden, schon nach einem halben Jahr Mikrofehler aufweisen, wobei ihre Anzahl mit der Zeit zunahm.

Die nach den Verfahren von Beispielen 1 bis 3 bearbeiteten Platten wurden ausserdem in Schenkelmuskeln von weissen Laborratten implantiert, wobei die Gewebe des Implantats dann morphologisch untersucht wurden. Diese Untersuchung hat ergeben, dass die nach dem angegebenen Verfahren bearbeiteten Titanplatten biologisch auf die Umgebungsgewebe praktisch nicht wirken.

- 7 -

## Industrielle Anwendbarkeit

Die vorliegende Erfindung lässt sich zur Herstellung von Herzklappenprothesen sowohl Aorten- als auch Mitralherzklappenprothesen mit Erfolg verwenden. Analog können ausserdem auch Röhrenelemente für den Ersatz von geschädigten Gefässen bearbeitet werden.

PATENTANSPRÜCHE

1. Titanimplantat für die kardiovaskuläre Chirurgie, welches bearbeitete thrombenresistente und biologisch träge Oberflächen aufweist, dadurch g e k e n n - z e i c h n e t, dass die bearbeiteten Oberflächen plastisch verformte und unter Einwirkung von freien granulierten Körpern verdichtete Oberflächen sind.

2. Verfahren zur Herstellung des Titanimplantats nach Anspruch 1, welches die Formung des Werkstücks für Implantat und die anschliessende Vor- und Nachbearbeitung seiner Oberfläche vorsieht, dadurch g e k e n n - z e i c h n e t, dass man im Stadium der Nachbearbeitung das Werkstück in eine Kammer, angefüllt mit freiem Medium in Form von polierten Granalien als Rotationskörpern und wässriger Seifenlösung, einbringt und die Kammer in Planetenbewegung mit einer Geschwindigkeit versetzt, die ausreicht, um die plastische Verformung des Werkstücks durch Zusammenwirken desselben mit Granalien auszulösen.

3. Verfahren nach Anspruch 2, dadurch g e k e n n - z e i c h n e t, dass Rotationskörper Kugeln darstellen.

4. Verfahren nach Anspruch 3, dadurch g e k e n n - z e i c h n e t, dass die Kugeln aus Chromstahl bestehen.

5. Verfahren nach Anspruch 4, dadurch g e k e n n - z e i c h n e t, dass die Kugeln einen Durchmesser von 0,8 bis 1,0 haben.

6. Verfahren nach Anspruch 5, dadurch g e k e n n - z e i c h n e t, dass die Kammer in Planetenbewegung mit einer zwischen 60 und 200 U/min liegenden Summengeschwindigkeit innerhalb von 2 bis 4 Stunden bei einem Kugeldurchmesser von 0,8 bis 1,0 mm versetzt wird.

EPAA-37822.3

0336933

| I. CLASSIFICATION OF SUBJECT MATTER (if several classification symbols apply, indicate all) 6 |
|---|

According to International Patent Classification (IPC) or to both National Classification and IPC

IPC⁴     A 61 F 2/24, A 61 L 27/00

| II. FIELDS SEARCHED |
|---|

| Minimum Documentation Searched 7 | |
|---|---|
| Classification System | Classification Symbols |
| IPC⁴ | A 61 F 2/24, A 61 L 27/00 |

Documentation Searched other than Minimum Documentation
to the Extent that such Documents are included in the Fields Searched 8

| III. DOCUMENTS CONSIDERED TO BE RELEVANT 9 | | |
|---|---|---|
| Category * | Citation of Document, 11 with indication, where appropriate, of the relevant passages 12 | Relevant to Claim No. 13 |
| A | US, A, 3911502 (JOHN W.BORETOS), 14 October 1975 (14.10.75), see claims | 1,2 |
| A | US, A, 4038702 (PHILIP NICHOLAS SAWYER), 2 August 1977 (02.08.77), see claims | 1,2 |
| A | US, A, 4101984 (DAVID C. MACGREGOR) 25 July 1978 (25.07.78)  see claims | 1,2 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

| IV. CERTIFICATION | |
|---|---|
| Date of the Actual Completion of the International Search | Date of Mailing of this International Search Report |
| 9 December 1988 (09.12.88) | 6 January 1989 (06.01.89) |
| International Searching Authority | Signature of Authorized Officer |
| ISA/SU | |

Form PCT/ISA/210 (second sheet) (January 1985)